# EUROPEAN PATENT APPLICATION

(11) **EP 4 332 233 A1**
(43) Date of publication of application: **06.03.2024**
(21) Application number: 22193833.5
(22) Date of filing: 05.09.2022
(51) Int. Cl.: C12P 17/02, C12N 9/20, C12R 1/72

(54) **SOLVENTLESS ENZYMATIC EPOXIDATION OF OLEFINS**

(71) Applicant: SYENSQO SA, 1130 Brussels (BE)
(72) Inventor: FERRãO, Victor, 13148-914 Paulinia (BR); MAZIERO, Priscila, 13148-914 Paulinia (BR)

(57) **Abstract**

The present invention relates to a method for enzymatic epoxidation of an olefin, the method comprising:
- combining:
an olefin,
using C5 to C12 carboxylic acid as at least one oxygen transfer agent in an amount of greater than zero and less than about 15% by moles, with respect to total moles of the olefin, and
an immobilized lipase enzyme which is Lipase B from Candida antarctica (Lipozym 435);
to form a mixture, and

- contacting the mixture at a reaction temperature with peroxide such as hydrogen peroxide as oxidizing agent in the absence of solvent, such that at least one epoxide is formed.

## Description

### Background

Generally speaking, the enzymatic epoxidation of olefins is a known process. See, e.g., EP 100 119, EP 230 949, WO 99/01445 and EP-A 659 473, to name a few. However, such processes use significant amounts of solvent, e.g., organic solvent. The use of such solvent is not only environmentally impactful, but also makes recovery of the final product more difficult. In addition, the conversion of olefin to epoxide is often quite low, with unfavorable yield and epoxide selectivity.

### Summary

In view of the catalytic epoxidation art, it is an object of the present invention to provide a method for the epoxidation of olefins that provides exceptional yields and product selectivity, while minimizing, or even completely eliminating the need for a solvent.

Accordingly, in some aspects, the present teachings are drawn to methods for enzymatic epoxidation of an olefin. Such methods generally include:
- combining:
   an olefin,
   at least one oxygen transfer agent in an amount of greater than zero and less than about 15% by moles, with respect to total moles of the olefin, and
   an immobilized lipase enzyme;
   to form a mixture, and
- contacting the mixture at a reaction temperature with oxidizing agent in the absence of solvent, such that at least one epoxide is formed.

In some embodiments, the olefin is a compound of formula (I) 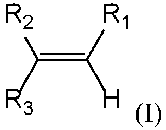 wherein
R1, R2 and R3 are each independently selected from an aromatic group or an aliphatic group, either of which may optionally comprise at least one heteroatom and may be optionally substituted with one or more aromatic, aliphatic, hydroxyl, ether, or amine groups,
or R1 and R2, together with the double bond, form a hydrocarbon ring, optionally substituted with one or more aromatic, aliphatic, hydroxyl, ether, or amine groups;
wherein the compound of formula (I) is liquid at or below the reaction temperature.

In some embodiments, the olefin is a compound of formula (I) 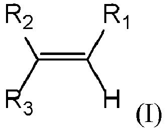 wherein
R1, R2 and R3 are each independently selected from an aromatic group or a C1-C10 aliphatic group, either of which may optionally comprise at least one heteroatom and may be optionally substituted with one or more aromatic, C₁-C₁₀ aliphatic, hydroxyl, ether, or amine groups,
or R1 and R2, together with the double bond, form a hydrocarbon ring, optionally comprising at least one heteroatom and optionally substituted with one or more aromatic, C₁-C₁₀ aliphatic, hydroxyl, ether, or amine groups.

In certain embodiments, the olefin is an optionally substituted cycloalkane, e.g., limonene.

In some embodiments, the oxidizing agent is an aqueous hydrogen peroxide solution, e.g., an aqueous solution comprising hydrogen peroxide in an amount of between about 30 wt% and about 50 wt%.

In some embodiments, the oxygen transfer agent is a carboxylic acid, e.g., a C₅-C₁₂ carboxylic acid. In certain embodiments, the oxygen transfer agent is octanoic acid.

In some embodiments, the reaction temperature is between room temperature and about 70°C, e.g., between about 30°C and about 60°C.

In some embodiments, the immobilized lipase enzyme is a lipase enzyme immobilized on a substrate in an enzyme amount of between about 1 wt% and about 20 wt%, relative to the total weight of the olefin .

In some embodiments, at least 50% of the olefin, e.g., at least 75% of the olefin, is converted to an epoxide.

### Brief Description of the Drawings

*Fig. 1* is a schematic representation of enzymatic olefin epoxidation.
*Fig.* 2 depicts the results of a kinetic study of the conversion of limonene to limonene oxide (recording limonene consumption) at 40°C (a) and 60°C (b), both over a 24 hour period.
*Fig.* 3 depicts the results of a kinetic study of the conversion of limonene to limonene oxide (recording limonene oxide production) at 40°C (a) and 60°C (b), both over a 24 hour period.
*Fig.* 4 depicts the maximum conversion, yield and selectivity for batch-type reactions performed with 0 wt%, 1 wt%, 2 wt%, 5 wt%, 7 wt%, and 10 wt% enzyme load at 40°C (a) and 60°C (b).

### Detailed Description

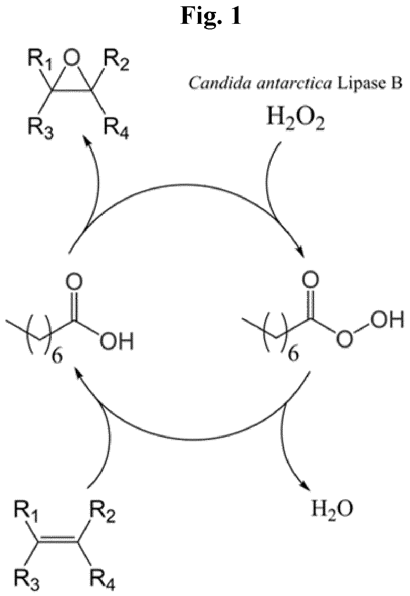

Disclosed herein is a *solventless* enzymatic epoxidation reaction, typically utilizing batch and fed-batch processes to oxidize an olefin to an olefin epoxide. Without wishing to be bound by any particular theory, it is believed that the olefin acts as a reducing agent in the presence an oxidizing agent, an oxygen transfer agent and a lipase catalyst. The lipase, which is immobilized on a substrate, catalyzes the formation of percarboxylic acids (or "peracids") from the carboxylic acids in the medium. The peracid species is then able to perform a Prilezhaev epoxidation reaction on the olefin, thus forming a corresponding epoxide. The peracid reverts to its original carboxylic acid, in a catalytic cycle as presented in Figure 1.

Accordingly, in some aspects, the present teachings are drawn to methods for enzymatic epoxidation of an olefin. Such methods generally include:
- combining:
   an olefin,
   at least one oxygen transfer agent in an amount of less than about 15% by moles, with respect to total moles of the olefin, and
   an immobilized lipase enzyme;
   to form a mixture, and
- contacting the mixture at a reaction temperature with oxidizing agent in the absence of solvent, such that at least one epoxide is formed.

In some embodiments, the contacting step comprises adding the oxidizing agent to the mixture in a single batch. In preferred embodiments, the contacting step comprises feeding the oxidizing agent to the mixture over a period of time. Suitable periods of time will generally depend upon the concentration of reagents and other reaction parameters, but can be, for example, from about 15 minutes to about 10 hours, e.g., from about 1 hour to about 5 hours.

In some embodiments, the mixture forms a bi-phasic system when contacted with the oxidizing agent. For example the oxidizing agent (e.g., hydrogen peroxide), is in an aqueous phase, whereas the olefin and the oxygen transfer agent (e.g., a carboxylic acid) are in an organic phase. Without wishing to be bound by any particular theory, it is believed that this simplifies the recovery process by maintaining the epoxide separate from the aqueous phase.

As used herein, the term "olefin" refers to an optionally substituted hydrocarbon having at least one double bond. Typically, olefins useful in connection with the present teachings are in liquid form at or below the reaction temperature. In some embodiments, olefins useful in connection with the present teachings are in liquid form at 60°C, e.g., at 50°C, at 40°C, at 30°C or even at 25°C or below.

In some embodiments, the olefin is a compound of formula (I) 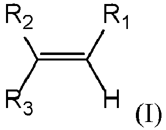 wherein
R1, R2 and R3 are each independently selected from an aromatic group or an aliphatic group, either of which may optionally comprise at least one heteroatom and may be optionally substituted with one or more aromatic, aliphatic, hydroxyl, ether, or amine groups,
or R1 and R2, together with the double bond, form a hydrocarbon ring, optionally substituted with one or more aromatic, aliphatic, hydroxyl, ether, or amine groups;
wherein the compound of formula (I) is liquid at or below the reaction temperature.

As used herein, the term "aliphatic" refers to a substituent having a straight or branched chain structure, wherein the chains comprise carbon atoms joined by respective single, double, or triple bonds. Aliphatic substituents may optionally be interrupted by one or more heteroatoms, typically selected from oxygen, nitrogen, and sulfur heteroatoms, and the carbon atoms may each optionally be substituted with one or more organic groups, typically selected from aromatic, aliphatic, hydroxyl, ether, or amine groups.

As used herein, the term "aromatic" refers to a substituent that comprises one or more cyclic, coplanar organic groups having a delocalized, conjugated π system, with a number of π electrons that is equal to 4n+2, where n is 0 or a positive integer. Aromatic substituents may include compounds where each of the ring members is a carbon atom, such as benzene or naphthalene, or compounds that include one or more heteroatoms, such as furan or pyridine, and the carbon atoms may optionally be substituted with one or more organic groups, typically selected from aromatic, aliphatic, hydroxyl, ether, or amine groups.

In some embodiments, the olefin is a compound of formula (I) 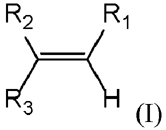 wherein
R1, R2 and R3 are each independently selected from an aromatic group or a C₁-C₁₀ aliphatic group, either of which may optionally comprise at least one heteroatom and may be optionally substituted with one or more aromatic, C₁-C₁₀ aliphatic, hydroxyl, ether, or amine groups,
or R1 and R2, together with the double bond, form a hydrocarbon ring, optionally comprising at least one heteroatom and optionally substituted with one or more aromatic, C₁-C₁₀ aliphatic, hydroxyl, ether, or amine groups.

In some embodiments, the olefin is a compound of formula (I) comprising at least 6 carbon atoms and at least one double bond. In some embodiments, the olefin does not comprise a bridging group.

In certain embodiments, the olefin is an optionally substituted cycloalkane. Optionally substituted cycloalkanes include, but are not limited to cyclohexane, 1-methyl-cyclohex-1-ene, 3-methylcyclohex-1-ene limonene, terpinen-4-ol, carvone, 2-carene, 3-carene, terpinolene, bisabolol. beta-phellandrene, In one embodiment, the olefin is limonene.

In other embodiments, the olefin is a substituted benzene. Substituted benzenes include, but are not limited to styrene, α-methylstyrene, eugenol, isoeugenol.

Other suitable olefins include, for example myrcene, citronellol, β-caryophyllene,linalool, nerol, geranlol, squalene, farnesene, alpha-ocimene, cis-beta-ocimene, trans-beta-ocimene, geraniol, aliphatic unsaturated hydrocarbons like: hexane, octane, decene, dodecene, and its suitable structural isomers.

The methods of the present teachings make use of an oxidizing agent. As used herein, the term "oxidizing agent" refers to a chemical compound that transfers oxygen atoms in a chemical reaction. In a general sense, the oxidizing agent should not be limited, and a person of ordinary skill in the art should be able to determine a suitable oxidizing agent for use in an epoxidation reaction with a lipase catalyst. However, in some embodiments, the oxidizing agent is a peroxide, such as hydrogen peroxide or tert-butanol hydroperoxide. In some embodiments, the oxidizing agent is an aqueous hydrogen peroxide solution. While the specific concentration of hydrogen peroxide in the solution may not be crucial, it should be concentrated enough to react with the other reagents, but not so concentrated so as to poison the catalyst. Aqueous solutions comprising hydrogen peroxide in an amount of between about 30 wt% and about 50 wt% are commonly available and may be preferred.

When hydrogen peroxide is used as the oxidizing agent, the natural byproduct is water. Accordingly, in certain embodiments, the method further includes removing water from the mixture. Water can be removed using a number of methods known in the art, the simplest of which is evaporation of the water and its collection, *e.g.,* in a Dean-Stark apparatus. Without wishing to be bound by any particular theory, it is believed that the removal of water from the system can shift the equilibrium towards the products.

The methods of the present teaching also make use of an oxygen transfer agent. As used herein the term "oxygen transfer agent" refers to a chemical compound that is capable to be catalytically oxidized into a transient species "accepting" an oxygen atom, so as to transfer it to an oxygen-recipient (in this case an olefin) in a chemical reaction, resulting in a oxidized chemical, plus the aforementioned "oxygen atom transfer". In the hereby method, the "oxygen atom transfer" is catalytically oxidized by the presence of the enzymes (e.g. the lipases). In some embodiments, enzymes used are of the class Esterases. In certain embodiments, the enzyme used is a Lipase. In some embodiments, the oxygen transfer agent is a carboxylic acid. Suitable carboxylic acids include, but are not limited to acetic acid, propanoic acid, butanoic acid, pentanoic acid, hexanoic acid, octanoic acid, decanoic acid, dodecanoic acid, tetradecanoic acid, and hexadecanoic acid. It is understood that smaller carboxylic acids could poison an enzyme catalyst and larger carboxylic acids might severely slow the kinetics of the reaction. A person of ordinary skill in the art would be able to select an appropriate carboxylic acid depending on other attributes (temperature, pressure, etc.) of the reaction. In some embodiments, however, the oxygen transfer agent is a C₅-C₁₂ carboxylic acid. In certain embodiments, the oxygen transfer agent is octanoic acid. In some embodiments, the carboxylic acid is liquid at or below the reaction temperature, or is soluble in the reaction solution.

In some embodiments, the amount of oxygen transfer agent used in the present method is between about 1 wt% and about 7 wt%, e.g., between about 2% and about 5%.

Although the reaction temperature is not particularly limited, it should be a temperature that does not adversely affect the enzyme and, preferably, is below the flash point of the substrate, while preferably allowing the olefin and oxygen transfer agent to remain either solubilized or in liquid form. Particularly suitable reaction temperatures are between room temperature and about 70°C. Excellent results have been achieved at temperatures between about 30°C and about 60°C, and particularly at 40°C. In some embodiments, the reaction temperature can be at or above the flash point. In such embodiments, undesirable effects (e.g. substrate combustion) can be mitigated by running the reaction in an inter atmosphere include, but limited to, nitrogen gas.

The lipase enzymes utilized in connection with the present application are immobilized on a substrate. Lipases are versatile enzymes, but soluble lipases are unstable and difficult to recover and reuse. Immobilization of the lipase increases its stability and facilitates its recovery and reuse. Lipases may be isolated from any one of a number of known microorganisms and may be immobilized on any one of a number of substrates, both of which are known in the art. Suitable microorganisms that may be a source of lipase include, but are not limited to *Pseudomonas, Geotrichum, Candida antarctica, Candida rugosa, Aspergillus, Rhizophus, Mucor hirmalis f. hiemalis*, *Streptomyces rimosus,* and *Penicillinum.* Suitable substrates for immobilization of lipase include, but are not limited to organic substrates such as polymers (thermoset or thermoplastic), and non-organic substrates such as aluminium oxide, silica and porous glass. Lipase may be immobilized on a substrate, for example, in an amount of between about 1 wt% and about 20 wt%, e.g., from about 1 wt% to about 10 wt%, e.g., between about 3 wt% and 7 wt%, relative to the weight of the substrate.

The methods of the present invention are not necessarily limited by an amount of immobilized lipase, however in general a greater amount of enzyme in the reaction tends to produce a greater conversion of olefin to epoxide. In some embodiments, the immobilized lipase is used in an amount of between about 1 wt.% and about 10 wt.%, relative to the amount of olefin.

The methods according to the present invention provide unexpectedly superior yield and selectivity. Typically, the methods of the present teachings provide at least 50% conversion of the olefin to an epoxide. However, in some embodiments, at least 55%, 60%, 65% or even 70% of the olefin is converted to an epoxide. In certain embodiments, at least 75% of the olefin is converted to an epoxide.

The invention will be described in greater detail by way of specific examples. The following examples are offered for illustrative purposes, and are not intended to limit the scope of the invention in any manner. Those of skill in the art will readily recognize a variety of noncritical parameters which can be changed or modified to yield essentially the same results.

### Exemplification

### Materials

(R)-(+)-Limonene (97% purity), Cyclohexene (99% purity), α-Methylstyrene (99% purity with 15 ppm of p-tert-butylcatechol as stabilizer), Eugenol (99% purity) and (1R)-(+)-α-Pinene (97% purity) were obtained from Sigma-Aldrich. Hydrogen peroxide (IX 501, 50 wt% aqueous solution with stabilizers) was obtained from Peróxidos do Brasil - Solvay. Octanoic acid (98% purity) was obtained from Solvay. Lipozym 435 (NZ435), lipase B from *Candida antartica* immobilized on a macroporous acrylic resin, was obtained from Novozymes A/S and was used as the biocatalyst. All reagents were used without further purification.

### Characterization

### Limonene oxide

Identification of limonene, limonene oxide and main byproducts was performed using the equipment Agilent HP7890B - G3440BA - CN16163098 with automatic injector 7693 and DB-WAXetr polar column (60 m × 0.32 mm × 1.0 µm). Samples were diluted in toluene together with n-amyl alcohol as internal standard and submitted to a heating ramp as follows: starting at 50 °C, heating at rate of 4 °C/min to 220 °C and maintaining to the last temperature for 17.5 min. Helium was used as carrier inert gas. Temperatures of injector and detector were 230 and 250 °C, respectively.

### Cyclohexene oxide

Identification of cyclohexene, cyclohexene oxide and main byproducts was performed using the equipment Agilent HP7890-MSD HP 5975C with HP5-MS (30 m × 0.25 mm × 0.25 µm) column. Quantification of compounds was performed using GC-FID with HP5-HT (same dimensions) column. 0,15 g of each sample was diluted in 0.8 mL of acetonitrile and 0.0040 g of internal standard 1,4-dioxane. Calibration curve was constructed with the concentrations 0, 100, 500, 1000, 2500 and 5000 ppm and internal standard was added with a concentration of 10 000 ppm for every concentration of the curve. Helium was used as the carrier inert gas. 0,5 µL of diluted sample was injected at 250°C and then it was submitted to a heating ramp as follows: starting at 45°C for 5 min, heating at a rate of 6°C/min to 270°C and maintaining at the last temperature for 10 minutes. The retention times observed were (in minutes): cyclohexane 2.21, cyclohexene 2.39, cyclohexene oxide 6.15; 2-cyclohexen-1-ol 7.19; 2-cyclohexen-1-one 8.67; 2-hydroxycycloexanone 10.67; 1,2-cyclohexanedione 11.17; 1,2-cyclohexanediol 12.76. The proportions (in percentage) of compounds in the organic phase were determined by the ratio of the area underneath one peak to the total area of the peaks.

### α-Methylstyrene oxide

The procedure was the same used in cyclohexene oxide analysis. Retention times observed were (in minutes): α-methylstyrene 10.62, acetophenone 12.96, α-methylstyrene oxide 13.57, 2-phenylpropanal 13.93, 2-phenylprop-2-en-1-ol 17.25, 2-phenylpropane-1,2-diol 19.18.

### Eugenol oxide

The procedure was the same used in cyclohexene oxide analysis. Retention times observed were (in minutes): eugenol 20.49, dihydroeugenol 22.28, guaiacylacetone 24.60, eugenol oxide 24.81, vanillyl glycol 28.76, hydroferulic acid 27.42.

### α-Pinene oxide

The procedure was the same used in cyclohexene oxide analysis and methanol was used instead of acetonitrile. Retention times observed (minutes): camphene 9.06, camphenol 13.60 and 14.15 (isomers); beta-pinene 9.95, α-phellandrene 10.82, α-campholenaldehyde 13.39, 14.35 and 21.20 (isomers); levoverbenone 16.51, *cis*-Carveol 16.77. α-Pinene oxide was not detected.

### Example 1: Synthesis of Olefin Epoxides: Batch Process

### 1A - Synthesis of limonene oxide

In three 50 mL penicillin amber flasks, the following amounts of components were added: 18.3612 g of limonene (0.131 mol), 0.6633 g of octanoic acid (0.0045 mol), 0.8905 g of NZ435 (5wt% based on cyclohexene mass) and, lastly, 8.8901 g of hydrogen peroxide solution (0.131 mol). The result was a 30 mL biphasic system consisting of an organic layer (top) and an aqueous layer (bottom). A blank reaction (with the absence of enzyme) was also performed. The flasks were sealed and taken to an incubator at 40 °C with orbital mixing and nitrogen to establish an inert environment. Reactions occurred for 24 h. Sampling of organic layer was performed by collecting 500 µL of it at the following reaction times: 0, 2, 4, 6 and 24 h. GC/MS analyses were conducted and the conversion of substrate and production of its oxide were determined, as well as main byproducts formed.

### 1B - Synthesis of cyclohexene oxide

The same procedure was used, except with cyclohexene instead of limonene, with the following masses: 15.0876 g of cyclohexene (0.182 mol), 0.9227 g of octanoic acid (0.0063 mol), 0.7468 g of NZ435 (5wt% based on cyclohexene mass) and 12.3654 g of hydrogen peroxide solution (0.182 mol).

### 1C - Synthesis of α-methylstyrene oxide

The same procedure was used, except with α-methylstyrene instead of limonene, with the following masses: 18.4603 g of α-methylstyrene (0.155 mol), 0.7846 g of octanoic acid (0,0053 mol), 0,9138 g of NZ435 (5wt% based on α-methylstyrene mass) and 10.5157 g of hydrogen peroxide solution (0.155 mol).

### 1D - Synthesis of eugenol oxide

The same procedure was used, except with eugenol instead of limonene, with the following masses: 22.8126 g of eugenol (0.138 mol), 0.6979 g of octanoic acid (0.0047 mol), 1.1292 g of NZ435 (5wt% based on eugenol mass) and 9.3529 g of hydrogen peroxide solution (0.138 mol).

### 1E - Synthesis of α-pinene oxide

The same procedure was used, except with α-pinene instead of limonene, with the following masses: 18.1646 g of α-pinene (0.131 mol), 0.6657 g of octanoic acid (0.0045 mol), 0.8937 g of NZ435 (5wt% based on α-pinene mass) and 9.6442 g of hydrogen peroxide solution (0.131 mol).

### Results

The batch reactions were tested using the following ratio of components: substrate/hydrogen peroxide = 1 mol/mol, octanoic acid/substrate = 0.03 mol/mol and 5wt% of enzyme based on substrate mass. Table 1 shows the concentrations of substrates and its oxides observed from GC/MS analyses for triplicate reactions. Blank reactions (not shown) confirmed that the enzyme was necessary to observe any significant concentration value of oxide for every substrate.

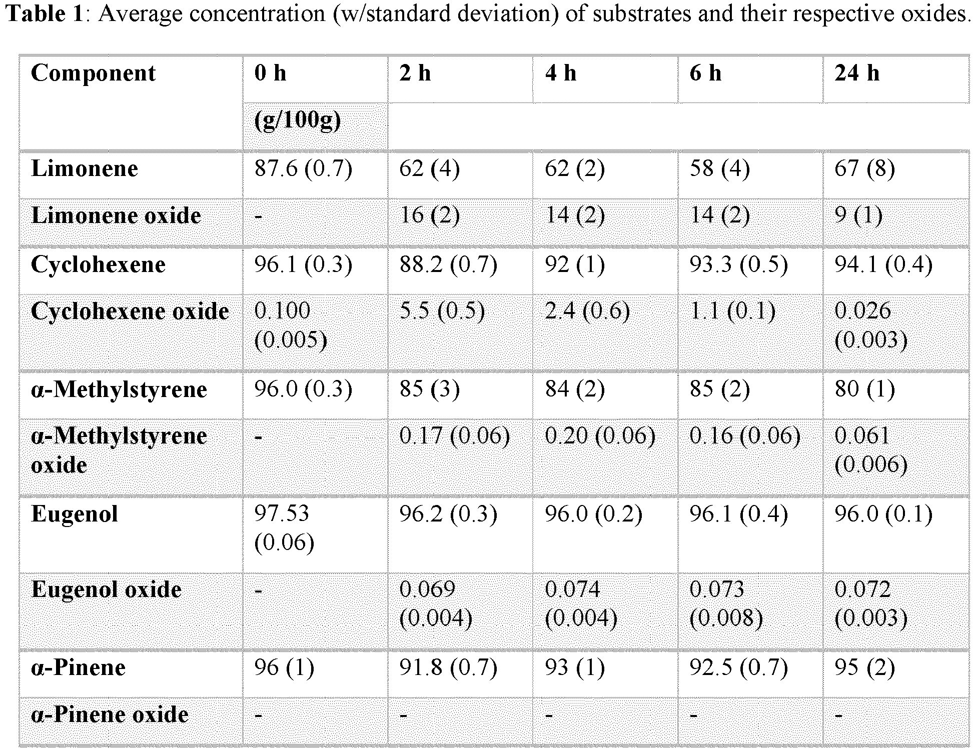

### Example 2: Synthesis of Olefin Epoxides: Fed-Batch Process (1)

### 2A - Synthesis of limonene oxide

In a three neck 50 mL jacketed glass reactor, 19.0714 g of limonene (0.136 mol), 0.6890 g of octanoic acid (0.0047 mol) and 0.9250 g of NZ435 (5wt% based on limonene mass) were added. A condenser was coupled to one neck, where water supply was maintained at 4-5 °C to it, and water jacketed was set to 40 °C. One end of silicon tubing was connected to one neck and the other end was placed in a glass bottle, filled with 9.2340 g of hydrogen peroxide solution (0.136 mol). The tubing was attached to a peristaltic pump and the remaining neck was sealed. The pump rate was maintained at 0.1 rpm (0.103 g/min estimated of hydrogen peroxide solution rate addition). Magnetic stirring was used, using a stirring bar. The reaction was then started when the first drop of hydrogen peroxide solution reached the reaction media. A biphasic system was formed composed by an organic layer and an aqueous layer as the reaction proceeded. Hydrogen peroxide was added for 1.5 h and the reaction time was 4 h. Sampling of the organic layer was performed by collecting 500 µL of it in 0, 0.75 h, 1.5 h and 4 h of reaction time. GC/MS analyses were conducted and the conversion of substrate and production of its oxide were determined, as well as main byproducts formed.

### 2B - Synthesis of cyclohexene oxide

The same procedure was used, except with cyclohexene instead of limonene, with the following masses: 11.2668 g of cyclohexene (0.136 mol), 0.6890 g of octanoic acid (0.0047 mol) and 0.5577 g of NZ435 (5wt% based on cyclohexene mass) and 9.2340 g of hydrogen peroxide solution (0.136 mol) were added.

### 2C - Synthesis of α-methylstyrene oxide

The same procedure was used, except with α-methylstyrene instead of limonene, with the following masses: 16.2102 g of α-methylstyrene (0.136 mol), 0.6890 g of octanoic acic (0.0047 mol), 0.8024 g of NZ435 (5wt% based on α-methylstyrene mass) and 9.2340 g of hydrogen peroxide solution (0.136 mol).

### 2D - Synthesis of eugenol oxide

The same procedure was used, except with eugenol instead of limonene, with the following masses: 22,5226 g of eugenol (0.136 mol), 0.6890 g of octanoic acic (0.0047 mol), 1,1149 g of NZ435 (5wt% based on eugenol mass) and 9.2340 g of hydrogen peroxide solution (0.136 mol).

### 2E - Synthesis of α-pinene oxide

The same procedure was used, except with α-pinene instead of limonene, with the following masses: 18.6861 g of α-pinene (0.136 mol), 0.6890 g of octanoic acic (0.0047 mol), 0.9250 g of NZ435 (5wt% based on α-pinene mass) and 9.2340 g of hydrogen peroxide solution (0.136 mol).

### Results

The ratio of components tested were substrate/hydrogen peroxide = 1 mol/mol, octanoic acid/substrate = 0.03 mol/mol and 5wt% of enzyme based on substrate mass. Table 2 shows concentrations in g/100g observed from GC/MS analyses, during the reaction.

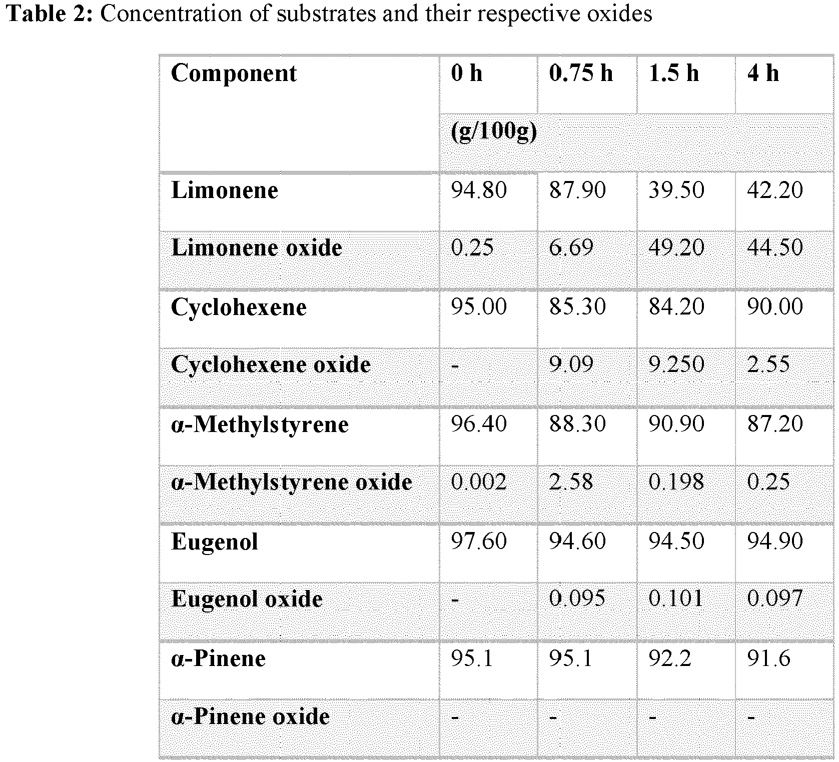

### Example 3: Synthesis of Olefin Epoxides: Fed-Batch Process (2)

### 3A - Synthesis of limonene oxide

In a four-neck 150 mL jacketed glass reactor, 50.0676 g of limonene (0.356 mol), 1.8311 g of octanoic acid (0.0127 mol) and 2.5089 g of NZ435 (5wt% based on limonene mass) were added. A Dean-Stark trap together with a condenser was coupled to one neck. An end of a silicon tube was connected to a metal tube and the last one was passed through a stopper, which was attached to one neck. Nitrogen bubbling was used to homogenize the reaction and disperse the enzymes in the medium by connecting the nitrogen supply tubing to a metal tube, which was passed to another stopper and this was attached to one neck. The remaining neck was sealed. A peristaltic pump was used as described in FB1 to add 23.7178 g of hydrogen peroxide solution (0.348 mol) and a biphasic system was formed during the reaction evolution. The peroxide addition time was estimated to 4 h and the reaction proceed for 5 h. Sampling of the organic layer (top) was performed at 0, 0.5, 1, 2, 4 and 5 h of reaction by collecting 500 µL of it. GC/MS analyses were conducted and the conversion of substrate and production of its oxide were determined, as well as main byproducts formed.

### 3B - Synthesis of eugenol oxide

The same procedure was used, except with eugenol instead of limonene, with the following amounts of components: 60.3013 g of eugenol (0.364 mol), 1.8447 g of octanoic acid (0.0125 mol), 2.9848 g of NZ435 (5wt% based on eugenol mass) and 24.7228 g of hydrogen peroxide solution (0.364 mol).

### 3C - Synthesis of α-pinene oxide

The same procedure was used, except with α-pinene instead of limonene, with the following amounts of components: 49.9722 g of α-pinene (0.363 mol), 1.8425 g of octanoic acid (0.0125 mol), 2.4736 g of NZ435 (5wt% based on α-pinene mass) and 24.6926 g of hydrogen peroxide solution (0.363 mol).

### Results

The ratio of components tested were substrate/hydrogen peroxide = 1 mol/mol, octanoic acid/substrate = 0.03 mol/mol and 5wt% of enzyme based on substrate mass. Table 3 shows concentrations in g/100g observed from GC/MS analyses, during the reaction.

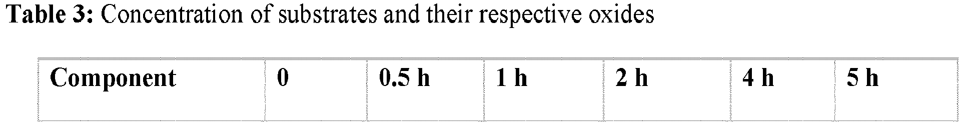

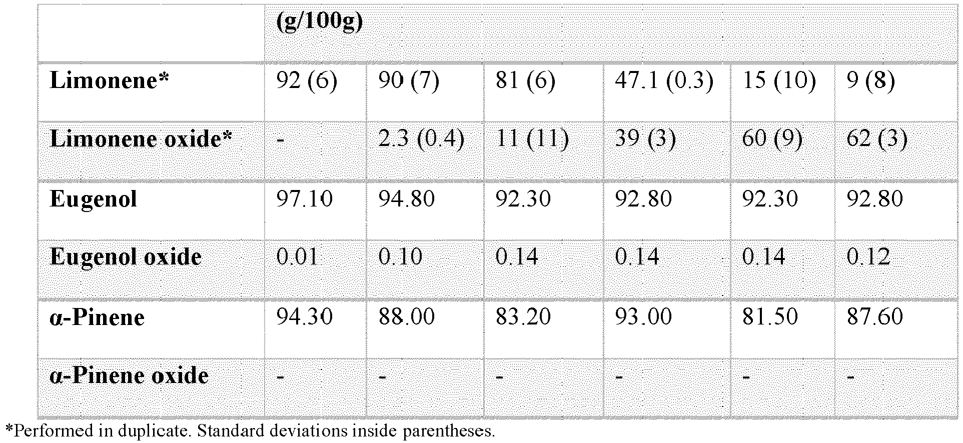

### Example 4: Synthesis of Olefin Epoxides: Fed-Batch Process (3)

### 4A - Synthesis of cyclohexene oxide

The procedure was the same as Example 3, except that mechanical stirring was used with mechanical stirrer. In addition, the ratio of substrate to hydrogen peroxide, the ratio of substrate to octanoic acid, the amount of enzyme based on substrate mass and the pump rate was changed (the last one to 0.2 rpm - 0.206 g of hydrogen peroxide solution added per minute). The following amounts of components were used: 29.6875 g of cyclohexene (0.358 mol), 10.5299 g of octanoic acid (0.0716 mol), 2.9391 g of NZ435 (10wt% based on cyclohexene mass) and 49.4536 g of hydrogen peroxide solution (0.716 mol).

### 4B - Synthesis of eugenol oxide

The same procedure was used, except with eugenol instead of cyclohexene, with the following amounts of components: 59.3386 g of eugenol (0.358 mol), 10.5286 g of octanoic acid (0.0716 mol), 5.8745 g of NZ435 (10wt% based on eugenol mass) and 49.4474 g of hydrogen peroxide solution (0.716 mol).

### 4C - Synthesis of α-pinene oxide

The same procedure was used, except with α-pinene instead of cyclohexene, with the following amounts of components: 49.2460 g of α-pinene (0.358 mol), 10.5318 g of octanoic acid (0.0716 mol), 4.8754 g of NZ435 (10wt% based on α-pinene mass) and 49.4626 g of hydrogen peroxide solution (0.716 mol).

### Results

Larger amounts of hydrogen peroxide, octanoic acid and enzyme were tested using the same configuration as Example 3: substrate/hydrogen peroxide = 0.5 mol/mol and octanoic acid/substrate = 0.2 mol/mol. Enzyme load was 10wt% based on substrate mass. Table 4 shows concentrations in g/100g observed from GC/MS analyses.

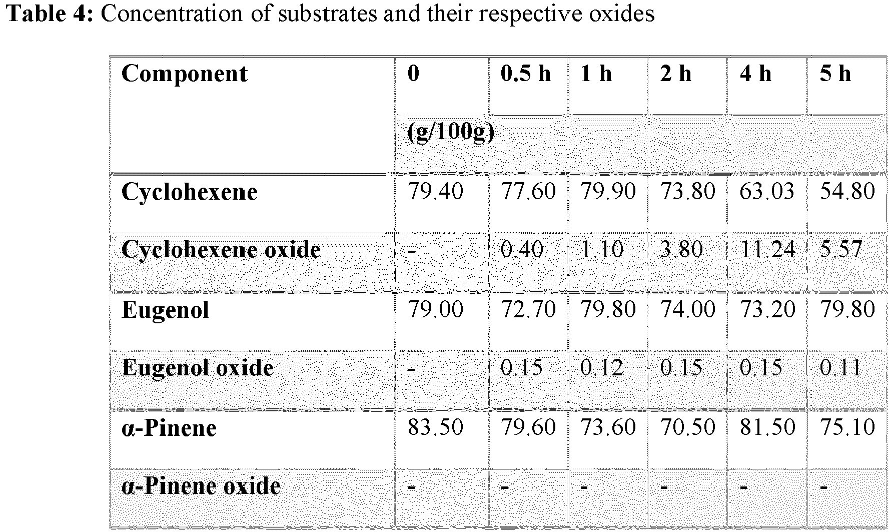

### Discussion of Examples 1-4

A comparison between data presented in Table 1 for batch reactions with data presented in Tables 2-4 for fed-batch reactions suggests that fed-batch process is typically more productive in terms of epoxide formation. Without wishing to be bound by any particular theory, it is believed that hydrogen peroxide causes damage to the enzyme and, by adding hydrogen peroxide slowly to the reaction media, its (and its peracid derivative's) time of contact with the enzyme is reduced.

Fed-batch (1) in Example 2 was performed with magnetic stirring, which was observed to damage the enzyme. Specifically, enzymes in contact with the substrates became mechanically weaker by swelling. According to Table 2, epoxide yields of up to 49%, 9%, 3% and 0.1% were observed for limonene, cyclohexene, α-methylstyrene and eugenol, respectively. Fed-batch (2) in Example 3 was performed with nitrogen bubbling in order to decrease mechanical damage to the enzyme. Applied to limonene and the other most challenging substrates (eugenol and α-pinene), the nitrogen bubble was effective to increase the yield of epoxide (up to 62% for limonene and 0.2% for eugenol). Therefore, the use of nitrogen to disperse the enzyme during the assay provided better limonene conversion and yield, as shown in a comparison between Examples 2 and 3. Switching from mechanical stirring to a dispersion caused by gas reduces the shear force upon enzymes, thus preserving their structure. In addition, and without wishing to be bound by theory, the gas may also remove excess water from the medium, potentially switching the equilibrium towards the products, hence the use of the Dean-Stark trap in Examples 3 and 4.

Fed-batch (3) in Example 4 was performed to determine the effect of molar ratio of enzyme, octanoic acid and hydrogen peroxide on the chemical reactions. While it was expected that increasing the amounts of oxidizing agent, oxygen transfer agent and biocatalyst may lead to increased yield, no significant increase in epoxide production was observed for the three species evaluated.

In all cases, limonene showed the highest epoxide production by enzymatic epoxidation. Limonene has three electron donor groups attached to a double bound, which provides a higher electron density and may be responsible for its greater reactivity. In Prilezhaev epoxidation, the more electron donor substituents a double bond possess, the more reactive it becomes. In addition, the double bond in limonene is not sterically hindered, which allows the reactive species (in this case, the peroxyacid formed from caprylic acid) to readily approach the double bond.

Cyclohexene provided the second highest production of epoxide, followed by α-Methylstyrene. Both of these molecules possess disubstituted double bonds, albeit the substitution occurs in different positions. α-Methylstyrene has a terminal double bond, thus its oxide is more susceptible to nucleophilic attack. While cyclohexene is still somewhat susceptible to such nucleophilic attack, having a substituent on each side of the double bond makes it less so. Diols (byproducts from hydratation) were detected in both cases, which confirms the susceptibility of these materials to nucleophilic attack.

Eugenol oxide was detected in trace amounts (<1wt%). It is believed that this may be due to the monosubstituted terminal double bond and phenolic group, which could have had a negative influence on the epoxidation. In addition, it is proposed that some dimerization or oligomerization of eugenol molecules occurred, as evidenced by the dark orange - red color of the reaction medium.

oxide formation was not observed in any case tested. Steric hindrance caused by the carbon bridge close to the double bond could be responsible for α-pinene's lack of reaction. As with eugenol, α-pinene may also have undergone dimerization or polymerization, as evidenced by the viscous and opaque reaction medium.

### Example 5: Influence of Temperature and Enzyme Load in Limonene Oxide Synthesis

Solventless limonene epoxidation was studied using six different proportions of lipase supported enzymes (LPZ435): 0 wt% (Blank), 1 wt%, 2 wt%, 5 wt%, 7 wt% and 10 wt%, at two different temperatures: 40°C and 60°C. The proportion of octanoic acid was not changed in any circumstance. The reactions were left for 24h.

A 50 ml amber flask was charged with the following reagents: D-Limonene 97%, octanoic acid 98%, LPZ435 from 0-10wt% (relative to the amount of limonene), and hydrogen peroxide 50%. Table 5 provides a summary of the reagent weights used in the experiments.

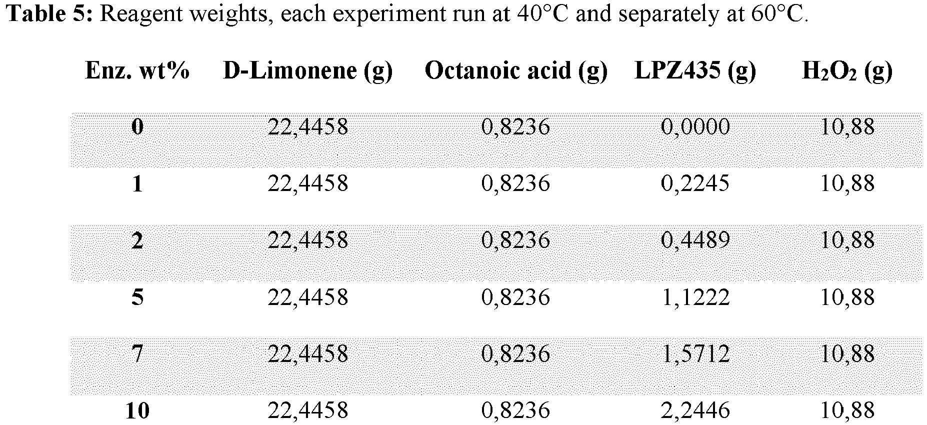

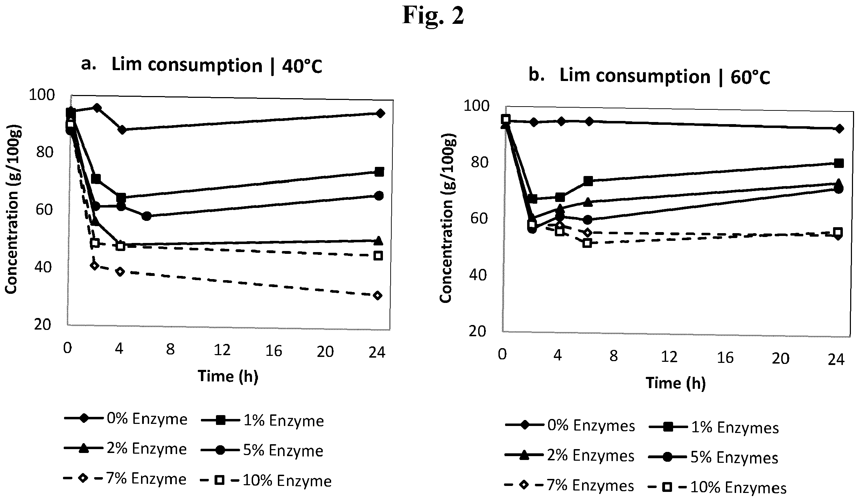

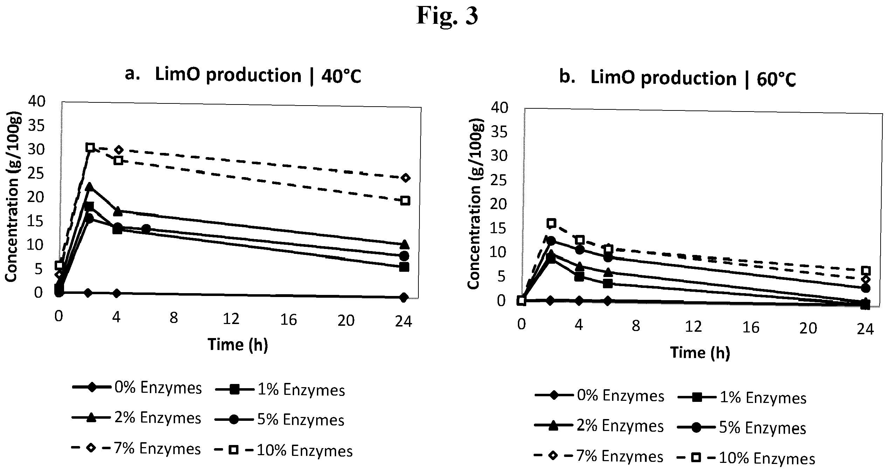

Limonene (Lim) consumption over time was measured at 40°C and 60°C for a period of 24 hours, and is summarized in Figure 2. Limonene monoxide (LimO) production over time was also measured at 40°C and 60°C for a period of 24 hours, and is summarized in Figure 3. As can be seen in Figures 2 and 3, a higher concentration of lipase enzyme tends to produce a greater consumption of Lim and a greater production of LimO. This trend is particularly obvious in the experiments performed at 60°C. However, it was also unexpectedly found that higher temperature lead to less conversion of Lim to LimO, although without significantly affecting the kinetics of the reaction. No conversion was observed in the experiments using 0wt% enzyme, validating the catalytic effect of the enzyme.

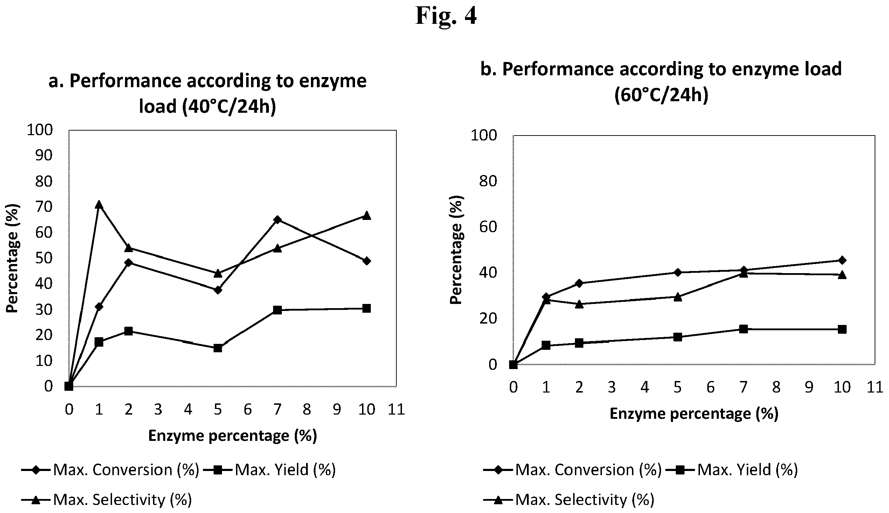

The results for maximum conversion, yield and selectivity were calculated from the kinetic studies for all enzyme loads, and are summarized in Figure 4. Again for both temperatures, the data confirms that no conversion occurs in the experiments using 0wt% enzyme. In addition, no optimum load of enzyme can be inferred from the results displayed in Figures 2-4, and no clear influence of the quantity of enzymes in the medium could be found for the kinetics of the reaction, at least in the first 2 hours. However, the kinetics from these batch-type assays show a common behavior regarding the consumption of Lim and production of LimO: a maximum consumption/production at between about 2 and 4 hours. After this time, the reaction appears to stagnate and LimO may undergo one or more parallel reactions.

## Claims

1. A method for enzymatic epoxidation of an olefin, the method comprising:
- combining:
an olefin,
at least one oxygen transfer agent in an amount of greater than zero and less than about 15% by moles, with respect to total moles of the olefin, and
an immobilized lipase enzyme;
to form a mixture, and
- contacting the mixture at a reaction temperature with oxidizing agent in the absence of solvent,
such that at least one epoxide is formed.

2. The method of claim 1, wherein the olefin is a compound of formula (I) 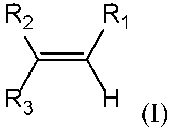 wherein
R₁, R₂ and R₃ are each independently selected from an aromatic group or an aliphatic group, either of which may optionally comprise at least one heteroatom and may be optionally substituted with one or more aromatic, aliphatic, hydroxyl, ether, or amine groups,
or R₁ and R₂, together with the double bond, form a hydrocarbon ring, optionally substituted with one or more aromatic, aliphatic, hydroxyl, ether, or amine groups; and
wherein the compound of formula (I) is liquid at or below the reaction temperature.

3. The method of claim 2, wherein the olefin is a compound of formula (I) 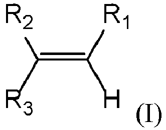 wherein
R1, R2 and R3 are each independently selected from an aromatic group or a C₁-C₁₀ aliphatic group, either of which may optionally comprise at least one heteroatom and may be optionally substituted with one or more aromatic, C₁-C₁₀ aliphatic, hydroxyl, ether, or amine groups,
or R1 and R2, together with the double bond, form a hydrocarbon ring, optionally comprising at least one heteroatom and optionally substituted with one or more aromatic, C₁-C₁₀ aliphatic, hydroxyl, ether, or amine groups.

4. The method of any one of the preceding claims, wherein the olefin is an optionally substituted cycloalkene.

5. The method of any one of the preceding claims, wherein the olefin is limonene.

6. The method of any one of the preceding claims, wherein the oxidizing agent is an aqueous hydrogen peroxide solution.

7. The method of any one of the preceding claims, wherein the oxygen transfer agent is a carboxylic acid.

8. The method of any one of the preceding claims, wherein the oxygen transfer agent is a Cs-C₁₂ carboxylic acid.

9. The method of any one of the preceding claims, wherein the oxygen transfer agent is octanoic acid.

10. The method of any one of the preceding claims, wherein the reaction temperature is between room temperature and about 70°C, e.g., between about 30°C and about 60°C.

11. The method of any one of the preceding claims, wherein the immobilized lipase enzyme is a lipase enzyme immobilized on a substrate in an enzyme amount of between about 1 wt.% and about 20 wt%, relative to the total weight of the olefin.

12. The method of any one of the preceding claims, wherein at least 50% of the olefin, e.g., at least 75% of the olefin, is converted to an epoxide.
